# EUROPEAN PATENT APPLICATION

(11) **EP 3 513 717 A1**
(43) Date of publication of application: **24.07.2019**
(21) Application number: 18152979.3
(22) Date of filing: 23.01.2018
(51) Int. Cl.: A61B 5/022, A61B 5/021, A61B 5/00, A61B 8/04, A61B 8/06

(54) **BLOOD PRESSURE MONITORING**

(71) Applicant: Koninklijke Philips N.V., 5656 AE Eindhoven (NL)
(72) Inventor: VAN HEESCH, Christianus Martinus, 5656 AE Eindhoven (NL); VAN HEESCH, Franciscus Hendrikus, 5656 AE Eindhoven (NL); DE WILD, Nico Maris Adriaan, 5656 AE Eindhoven (NL); PAULUSSEN, Igor Wilhelmus Franciscus, 5656 AE Eindhoven (NL); BEZEMER, Rick, 5656 AE Eindhoven (NL)
(74) Representative: de Haan, Poul Erik

(57) **Abstract**

A method of non-invasive, continuous monitoring of arterial blood pressure of a subject using a pressure cuff placed around a limb of the subject, comprises a periodic conventional oscillometric measurement of systolic and diastolic blood pressures. During these measurements, the arterial blood flow velocity is measured using Doppler ultrasound imaging. These measurements are processed and correlated. Between these direct measurements, unobtrusive monitoring is performed to check whether a direct measurement is needed, to avoid unnecessary direct measurements. The cuff pressure is set at a low level below the diastolic blood pressure, and is preferably slowly modulated at low levels. The arterial blood flow velocity waveform is continuously monitored, using the stored correlation, based on the Doppler ultrasound imaging.

## Description

### FIELD OF THE INVENTION

This invention relates to non-invasive and unobtrusive blood pressure monitoring.

### BACKGROUND OF THE INVENTION

There is increasing demand for unobtrusive health sensing systems. In particular, there is a shift from conventional hospital treatment towards unobtrusive vital signs sensor technologies, centered around the individual, to provide better information about the subject's general health.

Such vital signs monitor systems help to reduce treatment costs by disease prevention and enhance the quality of life. They may provide improved physiological data for physicians to analyze when attempting to diagnose a subject's general health condition. Vital signs monitoring typically includes monitoring one or more of the following physical parameters: heart rate, blood pressure, respiratory rate and core body temperature.

In the US about 30% of the adult population has a high blood pressure. Only about 52% of this population have their condition under control. Hypertension is a common health problem which has no obvious symptoms and may ultimately cause death, and is therefore often referred to as the "silent killer". Blood pressure generally rises with aging and the risk of becoming hypertensive in later life is considerable. About 66% of the people in age group 65-74 have a high blood pressure. Persistent hypertension is one of the key risk factors for strokes, heart failure and increased mortality.

The condition of the hypertensive patients can be improved by lifestyle changes, healthy dietary choices and medication. Particularly for high risk patients, continuous 24 hour blood pressure monitoring is very important and there is obviously a desire for systems which do not impede ordinary daily life activities.

Blood pressure is usually measured as two readings: systolic and diastolic pressure. Systolic pressure occurs in the arteries during the maximal contraction of the left ventricle of the heart. Diastolic pressure refers to the pressure in arteries when the heart muscle is resting between beats and refilling with blood. Normal blood pressure is considered to be 120/80 mmHg, where 120 is systolic and 80 is diastolic. A person is considered to be hypertensive when the blood pressure is above 140/90 mmHg, and two stages of hypertension may be defined for increasing blood pressure levels, with hypotensive crisis defined when blood pressure reaches 180/110 mmHg. Note that for conversion of these values to metric equivalents, 760.0 mmHg is equal to 101.325 kPa.

There are two main classes of method to monitor blood pressure.

For invasive direct blood pressure monitoring, the gold standard is by catheterization. A strain gauge in fluid is placed in direct contact with blood at any artery site. This method is only used when accurate continuous blood pressure monitoring is required in dynamic (clinical) circumstances. It is most commonly used in intensive care medicine and anesthesia to monitor blood pressure in real time.

For non-invasive blood pressure monitoring, NIBP, the traditional, manual method is the auscultatory method, using a stethoscope and a sphygmomanometer cuff. The cuff is placed around the upper arm at the height of the heart and is attached to a mercury or aneroid manometer. The cuff of the correct size for the patient is fitted smoothly and snugly and then inflated manually to completely occlude the artery. Listening with the stethoscope to the blood flow in the brachial artery near the elbow, the pressure is slowly released, and when blood starts flowing turbulently a "first Korotkoff sound" is heard, at the systolic pressure which is read from the manometer. The cuff pressure is released further until no sound can be heard, at the "fifth Korotkoff sound", which is at the diastolic arterial pressure, again read from the manometer.

This can be automated, and oscillometry is a popular automatic method to measure blood pressure. The method uses a similar pressure cuff with an integrated electrically controlled pressure sensor. The cuff is inflated and deflated automatically by a pump, whilst the pressure variations caused by the blood flow that changes over a heartbeat are sensed by the pressure sensor and are interpreted electronically to determine the phase of the heartbeat and thus the moments that the detected blood pressure is at its systolic and diastolic values. A microphone may also be used to detect the Korotkoff sounds which indicate the systolic and diastolic phases of the pressure variation.

The measurement from the pressure sensor is based on receiving the pulsated sounds of the blood passing the artery. Alternatively, the inhibition, obstructed and regular blood flow can be measured by using Doppler ultrasound: [NAVC2012] Procedures Pro/ NAVC Clinician's Brief/ May 2012 p26-30.

WO 2014/074901A discloses non-invasive, continuous, real-time monitoring of arterial blood pressure, using Doppler blood velocity measured using an ultrasonic transducer in a pressure cuff. The blood velocity variations are correlated with the sensed pressure variations to calibrate the system, so that subsequent velocity measurements alone can be used to monitor blood pressure, including estimated systolic and diastolic pressures. The calibration is repeated periodically. In an emergency, the cuff is used continuously for the pressure measurements at systolic and diastolic levels.

Some other non-invasive methods to estimate blood pressure are based on pulse wave velocity (PWV) rather than a Doppler measurement of velocity. This technique is based on the fact that the velocity of the pressure pulse traveling through an artery is related to blood pressure. The PWV is derived from the pulse transit time (PTT). Usually the pulse transit time is estimated by: (i) sampling and filtering the pressure waveforms, (ii) detecting beats in the waveforms, (iii) detecting features within the beats that serve as reference point (pulse arrival moment) and (iv) calculating the PTT as the time delay between the features.

PWV is obtained by: PWV = D/PTT, where D refers to the pulse travel distance. The PWV can be translated to a blood pressure estimate by means of a calibration step. Often the Moens-Korteweg equation is applied which describes the relationship between blood pressure and PWV. If the pulse travel distance D is stable during a measurement, the blood pressure can be directly inferred from PTT by means of a calibration function.

Blood pressure can also be measured by ultrasound without a cuff by determining both the velocity of the blood and the diameter of the blood vessel [Vosse 2011] Ultrasound in Med. & Biol., Vol. 37, No. 5, pp. 788-797, 2011. In this case the blood pressure is determined based on a model, which includes the elasticity of the arteries. The complexity of this measurement is relatively high due to the signal processing that is required and the accuracy is low due to the estimation of the elasticities. The velocity of blood in arteries can be determined accurately for a range of blood vessel diameters: [Hartley 2011] Am J Physiol Heart Circ Physiol 301: H269-H278, 2011.

Some other methods to measure blood pressure include techniques based on tonometry.

Currently automatic oscillometric blood pressure monitoring can be done using the wearable upper arm or ankle blood pressure cuff to perform periodic scheduled spot checks, for example once every half hour or every hour. These intermittent blood pressure measurements are usually too infrequent to detect rapid changes in blood pressure that can occur in acute care or during e.g. surgery situations. During these situations continuous monitoring using an invasive line is however not always appropriate, because it is invasive, poses an infection risk, or is problematic due to the patient being elderly, or because it is not available in emergency care, or it is not possible to use e.g. due to obesity. The non-invasive periodically-scheduled spot checks are not always necessary and they disturb the patient significantly, especially at night, or when at sleep during the day. On the other hand, since the spot-checks are scheduled and the cuff is unable to sense changes in vital signs when no pressure is applied, without invasive monitoring patient deterioration may be detected too late, increasing patient risk and cost of care.

There remains a need for a non-invasive blood pressure monitoring system that takes accurate measurements and is reliable for more continuous automatic monitoring yet minimizes the discomfort and disturbance of the patient.

### SUMMARY OF THE INVENTION

The invention is defined by the claims.

According to an aspect of the invention, there is provided a method of non-invasive, continuous monitoring of arterial blood pressure of a subject using a pressure cuff placed around a limb of the subject, comprising:
(a) measuring systolic and diastolic blood pressures whilst inflating and/or deflating the cuff between pressures at which arterial blood flow is impeded and at which it is unimpeded;
(b) measuring the arterial blood flow velocity using Doppler ultrasound imaging, whilst measuring the systolic and diastolic pressures;
(c) processing and correlating the measurements of blood pressure and blood flow velocity and storing a representation of their correlation;
(d) setting the cuff pressure at a low level which is below the diastolic blood pressure, and monitoring the arterial blood flow velocity waveform, using Doppler ultrasound imaging;
(e) determining from the monitoring in step (d) whether a predetermined trigger condition applies such that a pressure cuff-based measurement of blood pressure is required; and, if so,
(f) repeating step (a) to measure systolic and diastolic blood pressures. Preferably, step (f) comprises repeating steps (b) and (c) to recalibrate the stored representation of correlation.

The ultrasound imaging may be performed using a sensor embedded in a wearable e.g. an upper arm blood pressure cuff. It can be used to continuously measure heart rate and arterial flow without the application of significant cuff pressure, minimizing disturbance of the patient who may be asleep, and the discomfort of the high pressure. It can unobtrusively track/detect changes in heart rate and blood flow over time; and it can use these measurements of heart rate and blood flow to derive a surrogate (estimate) of blood pressure. A control system for the cuff can trigger or postpone regular or scheduled blood pressure measurements, so that they are made only when they are found to be necessary.

The low pressure level for the monitoring process is significantly lower than the obstruction or occlusion pressure, so that it is comfortable for the patient. There should still however be sufficient pressure to obtain reliable Doppler readings. The invention exploits the inherent advantage of ultrasound over pressure sensors or microphones, as it is able to sense the arterial pulses and flow rates in the absence of significant cuff pressure - enabling unobtrusive monitoring to be done between more obtrusive but more reliable pressure measurements.

The method preferably comprises in step (b) using a pressure sensor communicating with the cuff to measure the blood pressure oscillations due to the heartbeat. The pressure sensor may be external to the cuff, communicating with an air pump for the inflation of the cuff. Alternatively or in addition, the sensor may be integrated with the interior of the cuff, which is convenient and can also help the placement of the sensor over the artery, since the cuff typically has its own guides for facilitating correct placement.

The method preferably comprises using in step (b) an ultrasonic transducer in the cuff to measure the arterial blood flow velocity. Again, the integration of the transducer is convenient and can help its accurate placement over the artery to ensure strong transmission and reception of ultrasound pulses at the best angle to obtain the velocity data.

The method preferably comprises slowly modulating the low cuff pressure in step (d), with a modulation period preferably comprising a multiplicity of heartbeat periods, for example between 2 and 10, preferably between 3 and 7, most preferably five heartbeats, and using the Doppler ultrasound imaging to measure the arterial blood flow velocity waveform as it varies with this modulation. The modulation waveform is preferably sinusoidal. This causes the blood flow velocity to vary periodically, over successive heartbeats, between maximum and minimum values, and these extreme values vary with the pressure modulation.

The modulation of pressure provides more accurate predictions of the changes in blood pressure than are possible with a constant cuff pressure.

Accordingly, the method preferably comprises monitoring the variation, over successive periods of the slow modulation of the low cuff pressure, of the peak and/or minimum blood velocity in a heartbeat; and using the result of this monitoring to determine whether the predetermined trigger condition applies. The minimum pressure could in fact be zero, or close to zero, excess pressure (i.e. full deflation of the cuff, which will give rise to a pressure on the artery of atmospheric pressure affected only by any pressure caused by the fixing of the cuff). By way of example, the excess pressure on the arm caused by the fixing of the cuff could be 5% of the diastolic pressure.

For efficiency, the method preferably comprises monitoring the variation of the peak and/or minimum blood flow velocities occurring at the same phase of successive periods of the slow modulation. A substantial variation of values taken at the same phase over a number of cycles is indicative of a significant change of vital signs in the patient, so may be used to trigger a direct blood pressure measurement.

Conveniently, the method comprises scheduling the direct blood pressure measurements of steps (a) to (c) to occur regularly during each day and night, at intervals exceeding an hour (although the intervals could be set to be lower if the subject is at greater risk), preferably at intervals of three hours or more, and modifying that schedule either by eliminating some measurements or by adding previously unscheduled measurements in response to the determination in step (e) whether a direct measurement is required. This is an example of how the patient can be made more comfortable and allowed better sleep patterns, whilst still providing the safety of continuous monitoring of blood pressure and preferably other vital signs such as heart pulse rate.

In accordance with a further aspect of the invention, apparatus is provided for non-invasive, continuous monitoring of arterial blood pressure of a subject, comprising a pressure cuff arranged to be placed around a limb of the subject, means for measuring the pressure applied to the pressure cuff, and a Doppler ultrasound imaging system for imaging blood flow in the limb beneath or adjacent to the cuff, and a cuff controller configured to:
(a) measure systolic and diastolic blood pressures using the pressure measuring means whilst causing the inflation and/or deflation of the cuff between pressures at which arterial blood flow is impeded and at which it is unimpeded;
(b) measure the arterial blood flow velocity using the Doppler ultrasound imaging system, whilst measuring arterial blood pressure oscillations due to the heartbeat;
(c) process and correlate the measurements of blood pressure and blood flow velocity and store a representation of their correlation;
(d) set the cuff pressure at a low level which is below the diastolic blood pressure, and monitor the arterial blood flow velocity waveform, using the Doppler ultrasound imaging system;
(e) determine from the monitoring in step (d) whether a predetermined trigger condition applies such that a pressure cuff-based measurement of blood pressure is required; and, if so,
(f) repeat step (a) to measure systolic and diastolic blood pressures directly.

Preferably, step (f) comprises repeating steps (b) and (c) to recalibrate the stored representation of correlation.

This aspect has the advantages and the preferred features that correspond to those of the method of the first aspect.

Thus, preferably the cuff controller is configured in step (b) to use a pressure sensor communicating with the cuff to measure the blood pressure oscillations due to the heartbeat, and preferably an ultrasonic transducer in the cuff to measure the arterial blood flow velocity.

The apparatus is preferably configured to slowly modulate the low cuff pressure in step (d), with a modulation period preferably comprising a multiplicity of heartbeat periods, and to use the Doppler ultrasound imaging to measure the arterial blood flow velocity waveform as it varies with this modulation. Preferably it is configured for monitoring the variation, over successive periods of the slow modulation of the low cuff pressure, of the peak and/or minimum blood velocity in a heartbeat; and using the result of this monitoring to determine whether the predetermined trigger condition applies.

Preferably the apparatus is configured for monitoring the variation of the peak and/or minimum blood flow velocities occurring at the same phase of successive periods of the slow modulation.

Preferably, the apparatus is configured for scheduling the direct blood pressure measurements of steps (a) to (c) to occur regularly during each day and night, at intervals exceeding an hour, preferably at intervals of three hours or more, and modifying that schedule either by eliminating some measurements or by adding previously unscheduled measurements in response to the determination in step (e) whether a direct measurement is required.

In accordance with another aspect of the invention there is provided a computer program comprising computer program code means which is adapted, when said program is run on a computer of a cuff controller, to implement the method of the first aspect described above.

Features of examples and embodiments outlined in relation to one aspect of the invention, e.g. part of the processing method described above, may in advantageous embodiments be combined with or incorporated into any other aspect of the invention in further examples.

### BRIEF DESCRIPTION OF THE DRAWINGS

Particular embodiments of the invention will now be described in detail, by way of example only, with reference to the accompanying drawings, in which:
Fig. 1 is a schematic diagram of a wearable inflatable blood pressure cuff equipped with an ultrasound transducer;
Fig. 2 is a graph of cuff pressure P in mmHg against time T showing the linear variation of cuff pressure for sphygmomanometric measurement and the corresponding oscillometric waveform of pressure variations over successive heartbeats, as well as the amplitude of sound detected in a cuff microphone as it varies over time T, showing the Korotkoff sounds;
Fig. 3 is a graph corresponding to Fig. 2, also including graphs showing the waveforms, as the cuff pressure is relaxed from occlusion to full deflation, of blood vessel diameter D over time T, volumetric blood flow rate F over time T and blood flow velocity V over time T;
Fig. 4 shows a schedule for regular NIBP, non-invasive blood pressure, measurements over a day, with some measurements postponed and one previously unscheduled measurement;
Fig. 5 shows part of the schedule of Fig. 4, with an explanation of the triggering of the previously unscheduled measurement as a spot-check;
Fig. 6 shows a revised NIBP measurement schedule also including a graph of activity level of the patient measured using an accelerometer;
Fig. 7 shows two graphs of cuff pressure P and blood flow velocity V against time T;
Fig. 8 is a flow chart illustrating the application of an algorithm for the timing of blood pressure measurements;
Fig. 9 is a graph of cuff pressure P against time T showing scheduled direct measurements and an intermediate period of indirect monitoring; and
Fig. 10 is a schematic graph of blood flow velocity V against cuff pressure P illustrating the regions over which the velocity variations are monitored as shown in Fig. 9.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The preferred embodiments provide an ultrasound sensor embedded in a wearable (upper arm, or ankle) blood pressure cuff to:
continuously measure heart rate and arterial flow without application of significant cuff pressure;
unobtrusively track/detect changes in heart rate and flow over time;
use these measurements of heart rate and flow to derive a surrogate (estimate) of blood pressure; and
trigger or postpone regular blood pressure measurements based on the surrogate measurements.

This increases the comfort of the patient by postponing unnecessary spot-checks, especially at night, as well as the safety of the patient, by rapidly responding to changes in heart rate and/or blood flow.

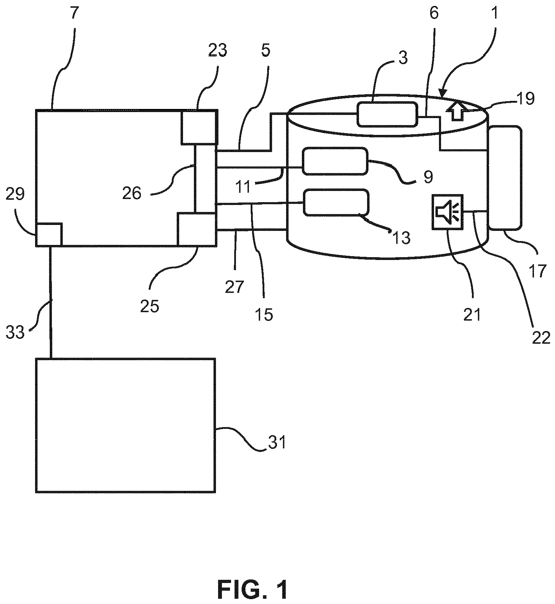

As shown in Fig. 1, a wearable inflatable blood pressure cuff 1 as a first embodiment of the invention is equipped with an integrated ultrasound sensor with an ultrasound transducer 3 for Doppler measurements of blood flow velocity in the artery beneath the cuff. The ultrasound sensor can either be a wide-angle single-element continuous-wave Doppler transducer, which can be made inexpensively but which requires more careful placement, or a 1D or 2D array where beam-steering can be used to optimize the measurement. This ultrasound transducer can be used instead of or in addition to the standard pressure sensor or microphone for registration of the arterial pressure pulses and Korotkoff sound, respectively. Ultrasound transducer signals 5 are communicated to a control unit 7 which controls the Doppler detection process to image the blood flow. The ultrasound transducer need not be permanently connected to the cuff; for example, it could be a separate module that is then replaceable. In one such example, the Doppler transducer comprises a handle including a USB connector, connected integrally to a flat probe which includes a Capacitive Micromachined Ultrasound Transducer, CMUT, mounted on a flex foil. The probe is positioned at the correct location (e.g on the arm) and is stuck to the skin using a skin adhesive in such a way that the transducer has the artery in view and adequate acoustic contact is made. The pressure cuff is mounted in the usual way on the limb, and is positioned over the probe, so that the end of the flex foil is beneath one edge of the cuff.

An optional microphone 9 integrated into the cuff provides sound amplitude signals 11 to the control unit 7 for detecting Korotkoff or other signals, for timing the direct systolic and diastolic pressure measurements and/or assisting in positioning the cuff and/or establishing the pattern of the heartbeat.

An optional accelerometer 13 integrated into the cuff provides activity signals 15 to the control unit 7 to enable the system to determine when the patient is at rest, perhaps sleeping, or is awake and moving, and optionally also to determine posture. This can help schedule the direct blood pressure measurements for the best comfort and least disturbance to the patient.

The design of the cuff guides the user for proper placement of the device. For example, in the device of Fig. 1 the cuff is equipped with a display 17, connected by line 6 to the ultrasonic transducer 3, which should be on the outside of the patient's arm (or ankle), and a mark such as an arrow 19 can be used to further guide the user, to align the cuff with the expected location of the brachial artery. The cuff may be equipped with a solid acoustic window (e.g., a hydrogel), but otherwise a liquid acoustic gel can be placed on the inside of the cuff at the location of the transducer 3. Applying minor pressure (only contact pressure; far below diastolic pressure) with the cuff ensures sufficient acoustic contact with the skin. In addition, the Doppler signal can be made audible, by the control unit 7 or the transducer 3 amplifying the ultrasonic echo signal and generating from it an acoustic signal driving a speaker 21 connected by line 22 to the screen 17 (or driving the cuff microphone 9 as a speaker) to guide the placement of the cuff; in this way the user can hear when the transducer is placed above an artery.

The cuff has a pressure sensor 23 which may be in the control unit 7 to communicate with an electric air pump 25 on line 26, the air pump 25 connected to the cuff over a pipe 27. The pressure sensor may be electronic, and may comprise a piezoelectric transducer. The air pump is controlled automatically to inflate and deflate the cuff to squeeze the limb and thus apply pressure to the artery, whilst the arterial pressure can be sensed by the pressure sensor 23. The pressure sensor may be part of the air pump 25. Alternatively, the pressure sensor may be closer to the cuff or even within the cuff.

The pressure sensor 23 provides a sufficiently accurate measure of the pressure level for the purposes of recording systolic and diastolic blood pressures, mean pressures and other measures; it is also useful for outputting a time-varying waveform showing pressure variations occurring cyclically with the heartbeat.

The more conventional aneroid manometer as a manually-operable pressure sensor may be included, connected in the usual way to the air pump and cuff. This will still need calibration periodically, but should be more accurate than piezoelectric sensors. It may be used to calibrate the automatic pressure sensor 23 periodically, for example by the user entering the reading at an interface of the control unit 7. A mercury manometer may also be used, for still greater accuracy.

The control unit includes a communications unit 29 for sending and receiving data to and from an external system 31 such as in a hospital. This transmission over a link 33 may be wired, e.g. to a monitoring unit next to a patient's bed, and/or wireless e.g. to a hospital medical system. The monitoring data may be stored locally in the control unit 7 for batch download, or the monitoring data may be provided in real time to the external monitoring unit. Many different possible approaches for storing data and providing to an external monitoring unit will be apparent to those skilled in the art.

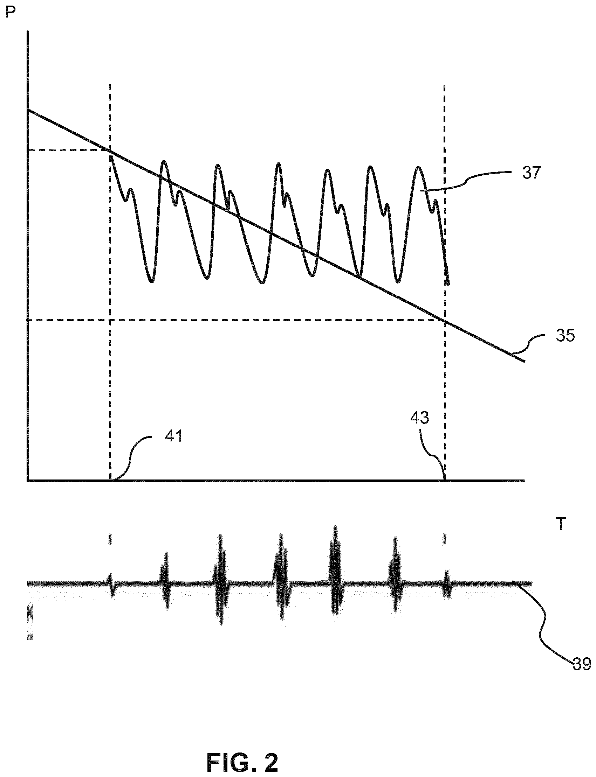

Once the cuff is placed correctly, the device starts a direct cuff-based blood pressure measurement by inflating the cuff to suprasystolic pressure and subsequently deflating the cuff, as shown by the line 35 in Fig. 2. Typically, during the inflation and deflation of the cuff, the device registers the arterial pressure pulses 37 sensed in the pressure sensor 23 and/or the Korotkoff sounds on an acoustic waveform 39 sensed on the microphone 9 and plots these against the cuff pressure 35, to find the systolic blood pressure at time instant 41, the diastolic blood pressure at time instant 43, and mean blood pressure.

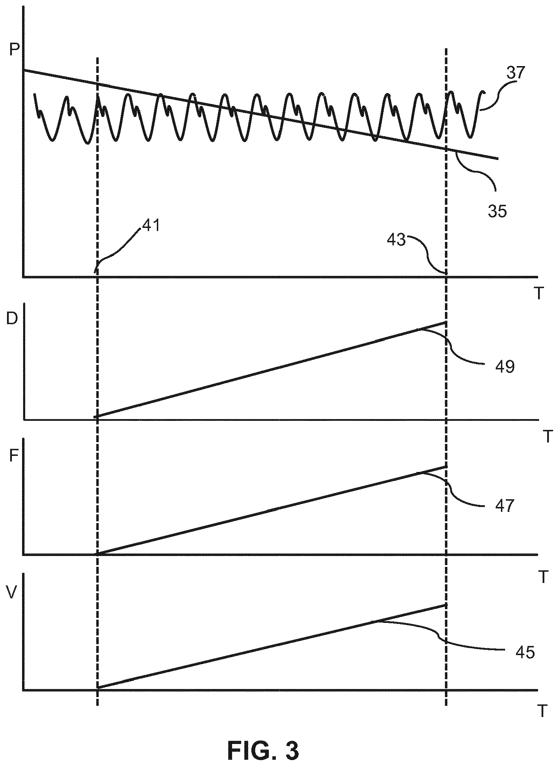

The microphone 9 is not necessary, as plotting the arterial flow against the cuff pressure also allows identification of diastolic pressure (i.e., the cuff pressure at which blood flow starts to reduce) and systolic pressure (i.e., the cuff pressure at which blood flow is reduced to 'a minimum'). A calibration curve should be constructed for this. As shown in Fig. 3, the control unit 7 calibrates the variation of blood flow velocity V over time T with the systolic blood pressure at moment 41 of time T and diastolic blood pressure at moment 43.

As a first approximation, as shown in Fig. 3, the blood flow velocity V, in graph 45, varies linearly with the cuff pressure P, over the range between systolic and diastolic pressures (between time instants 41 and 43). It is at a minimum close to the occlusion of the artery at systolic pressure and rises steadily to the diastolic pressure.

This linear variation can be recorded as a model of the variation of pressure with velocity, from which the pressure can be estimated from Doppler-measured velocity in lengthy periods between direct cuff-based measurements. However, preferably more parameters are used, and with greater precision, to determine the model from which pressure is estimated in these intervening periods.

The Doppler measurements can also be registered with the waveforms 47, 49 of blood volumetric flow F and blood vessel diameter D as shown in Fig. 3. As is well known, the diameter D varies between patients as a function of vessel elasticity. The diameter can be derived from the presence of Doppler signals in a PWD, pulsed-wave Doppler mode. This registration process is in effect a correlation of the variations between these variables, together with the correlation of blood pressure P and blood velocity V. The correlation process can be performed by the control unit 7 during the direct blood pressure measurements, and then repeated as a calibration with each successive direct blood pressure measurement. The models representing these variations can be stored in the control unit 7, for application during the intervening periods for the monitoring of vital signs including pressure and preferably pulse rate and volumetric flow rate. The correlation(s) may take the form of a computer model, a mathematical equation, or a numerical look-up table, stored in memory connected to a data processor within the control unit 7.

One of the advantages of using an ultrasound sensor rather than a pressure sensor or microphone is that an ultrasound sensor is able to sense the arterial pulses and flow, in the absence of significant cuff pressure, which is not possible with the other sensors. This allows unobtrusive monitoring of the heart rate and blood flow in between the obtrusive cuff-based measurements. So while cuff-based blood pressure measurements are still done intermittently, the device is now able to continue physiological monitoring and identify changes in hemodynamics at an early stage allowing timely intervention without adding any unnecessary burden to the patient.

After a cuff-based blood pressure measurement, the unobtrusively measured systolic and diastolic blood flows, the heart rate, as well as other features in the Doppler waveform (e.g., steepness of rising systolic flank) can be used to provide a surrogate measure of blood pressure via the stored computer model, mathematical equation, or look-up table, in the data processor within the control unit 7. Thus for example the velocity waveform V measured by the Doppler transducer is analyzed by the data processor and used to derive surrogate values of systolic and diastolic blood pressure from the mathematical model, equation or look-up table. Furthermore, the response of the flow measurements F to a reduction (and rise) in blood pressure P can be learned during the direct cuff-based blood pressure measurement; inflating the cuff lowers the blood pressure and flow downstream as shown graphically in Fig. 3. The response of the Doppler waveform to this is recorded and used to interpret changes in the signal in the absence of cuff pressure.

This monitoring process, between direct cuff-based measurements and recalibrations, is carried out at a relatively low cuff pressure, to minimize patient discomfort and disturbance, so that it can be continued for many hours without causing harm. For this reason, the cuff pressure is below diastolic pressure, but still sufficiently high to maintain its position on the patient's limb. The excess pressure from the cuff that is sufficient to maintain its position on the limb may for example be 5% of the diastolic pressure, i.e. about 5mmHg. Preferably it is between 95% and 5%, more preferably 50% to 10%, of the diastolic pressure. If for example the diastolic pressure is 100mmHg, then the cuff pressure is set to a value that is in the preferred range of 5mmHg to 95mmHg above atmospheric pressure, more preferably 10mmHg to 50mmHg above atmospheric pressure. The range could for example be set to a value within a predetermined pressure range such as 8 to 40 mmHg, i.e. 10 to 50% of a normal 80 mmHg diastolic pressure.

Since the ultrasound transducer 3 is able to provide a continuous measurement of flow, heart rate, and surrogate blood pressure, it can be used to postpone an unnecessary spot-check (reducing burden on the patient, especially at night) and to trigger a spot-check and/or an alarm when these parameters start to deviate or deteriorate. This increases patient safety, especially at night.

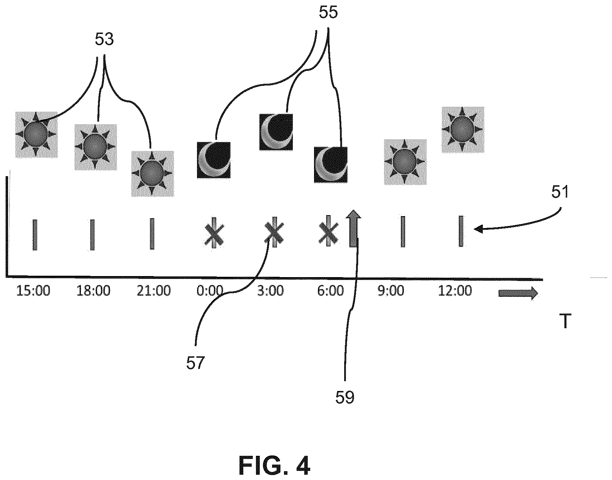

As shown in Fig. 4, direct measurements shown as vertical lines 51 beneath a sun 53 or moon 55 symbol are scheduled at intervals of 3 hours (this could be less, e.g. if the patient is considered at relatively high risk, or more). The crosses 57 over the lines 51 at midnight, 3am and 6am mean a postponed measurement, and the vertical arrow 59 at 7am indicates a trigger of an extra, previously unscheduled NIBP measurement.

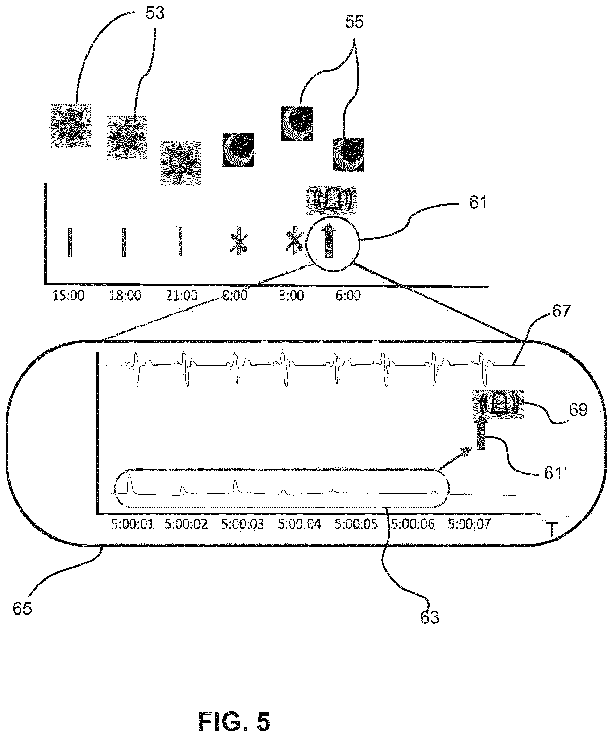

As shown in greater detail in another example of a schedule shown in Fig. 5, a trigger 61 occurs at 5am, generated by the control unit 7 from the ultrasound waveform 63. A display element 65 as shown for the trigger 61 includes the ultrasonic waveform 63, a pulse waveform 67, a vertical arrow 61' and a bell icon 69. This also triggers an audible alarm as indicated by the icon 69 adjacent the vertical trigger line 61'. The need for an audible alarm is assessed according to a predetermined algorithm stored in the control unit 7, based on the characteristics of the waveform 63 indicative of changes in vital signs. This follows two postponed scheduled measurements 51 at midnight and at 3am.

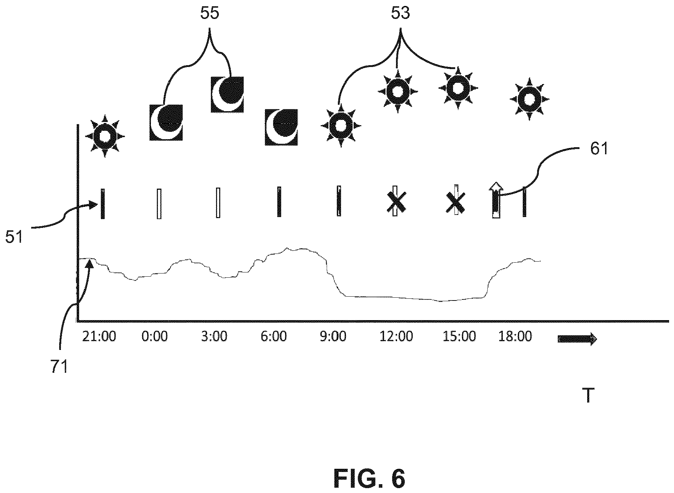

Patients in hospital can have a disturbed day and night rhythm which results in them sleeping during the day. To adapt to this and to support more accurate triggering, the patient's heart rate can be analyzed from the Doppler waveform, to determine whether the patient is asleep and should not be disturbed unless necessary. The optional accelerometer 13 could be used, as shown in Fig. 6, to detect patient activity represented by waveform 71 and/or arm position, or more generally the patient's posture. This also can help to postpone direct NIBP measurements 51 when the patient is sleeping or is recumbent for other reasons during day time.

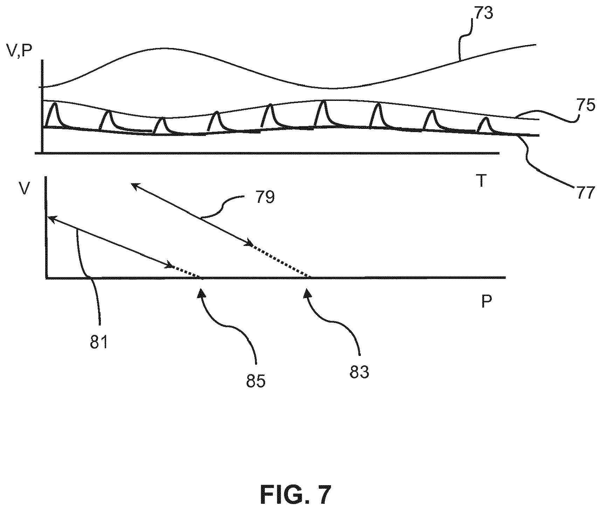

A second embodiment of the invention, which is a variation of the first embodiment, will now be described with reference to Figs. 7 to 10. Only the differences will be described in detail.

As shown in Fig. 7, after the direct blood pressure measurement, the blood velocity V is continuously monitored over time T by Doppler ultrasound recording the maximum and minimum blood velocity during the heartbeat cycle. In order to get a more accurate prediction of changes in blood pressure than is possible with the cuff pressure staying constant and low, as described above with the first embodiment, in this second embodiment the comparison of the blood velocity changes is implemented with a modulating cuff pressure 73. The pressure of the cuff is slowly modulated between two relatively low pressures, which are below the diastolic pressure. The lower of the pressures could be close to full cuff deflation. As indicated above, this lower of the two low pressures should be sufficient to maintain the position of the cuff on the limb and it may for example be 5% of the diastolic pressure. The higher pressure is still lower than the diastolic pressure, but high enough significantly to reduce the average (and also the maximum 75 and minimum 77) blood velocity V. In Fig. 7, the slowly modulated cuff pressure P is the top line 73 of the upper graph, and the blood velocity V is the pulsed waveform of the lower part of the upper graph which varies between the maximum 75 and minimum 77. Minimum and maximum blood velocity values are obtained at specific phases of the cuff pressure modulation. The velocity values obtained at the same phase but in successive modulation cycles are compared. A significant velocity difference could indicate a change in blood pressure and therefore a new direct blood pressure measurement is triggered by fully inflating and deflating the cuff, followed again by the slow pressure modulation. In this way, blood pressure is measured directly only when it is changed, the changes measured in the surrogate or estimated values over time.

The modulation period comprises a multiplicity of heartbeat periods, for example between 2 and 10, preferably between 3 and 7, most preferably five heartbeats. Since the advantages derive from variation of the pressure, in principle any form of variation is technically feasible. The variation need not be a smooth modulation, although this is beneficial as it avoids disturbance of the flow by abrupt changes of pressure. The waveform of the modulation need not be regular and need not be symmetrical between its peak and its trough - it could for example involve a longer duration at higher pressures than at lower pressures.

The same maximum and minimum blood velocities 75 and 77 are expressed in the lower graph of Fig. 7 respectively as variations 79 and 81 as a function of cuff pressure P, which occur over monitoring values 83 and 85 of pressure P. The minimum blood velocity varies with pressure P over the range shown between the arrows on the line 81, this range of minimum values being the same as the range of the line 77 which expresses the variation as a function of time T instead of pressure P. Correspondingly, the maximum blood velocity varies over the range 79 which is at higher pressures P, the range being the same as the range of the line 75.

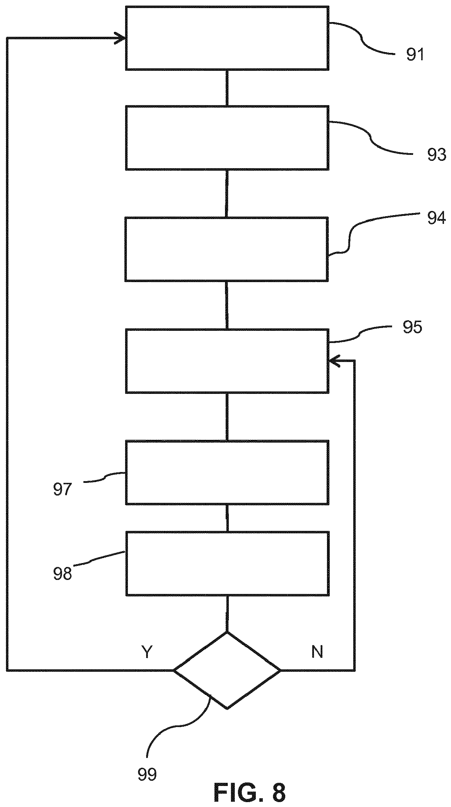

Fig. 8 illustrates a processing flow chart, performed by the data processor in the control unit 7, using an algorithm for the blood measurement procedure. The method involves taking a decision as to when to trigger the start of a regular direct blood pressure measurement.

Initially in step 91, systolic and diastolic pressures are measured by the cuff in the regular, direct way by inflating and/or deflating the cuff between pressures at which arterial blood flow is impeded and at which it is unimpeded.

In step 93 the arterial blood flow velocity is measured using Doppler ultrasound imaging, whilst measuring the systolic and diastolic pressures. Thus, steps 91 and 93 occur concurrently.

In step 94, the measurements of blood pressure and blood flow velocity are correlated and a representation of their correlation is stored.

This step of generating a correlation may take place after each cuff based measurement, but it may be performed less frequently. Thus, step 94 is optional and may be performed only in some cycles.

In step 95, the cuff pressure is set to at a low level substantially closer to diastolic than systolic blood pressure. In step 97, the pressure is then modulated between minimum and maximum pressures, preferably sinusoidally, although other variation patterns are of course feasible, as described above. The arterial blood flow velocity waveform is monitored, using Doppler ultrasound imaging. Doppler velocity measurements are taken and stored, determining the maximum 75 and minimum 77 blood velocity V occurring at a specific phase of the pressure modulation cycle. This may be repeated for other specific phases.

Next, in step 98, the maximum values of V obtained at the same phase in successive cycles are compared, and/or the minimum values of V obtained at the same phase in successive cycles are compared.

In step 99 it is determined whether a predetermined trigger condition applies such that a pressure cuff-based measurement of blood pressure is required. In particular velocity difference values over time (of the minimum and/or maximum velocity values) are compared in step 99 with predetermined threshold values to determine whether they are significant. Sudden changes in maximum and minimum velocity during cuff pressure modulation are used to act as a trigger. If so, the method returns to step 91 so that the step of measuring systolic and diastolic blood pressures using cuff pressure control is repeated.

Optionally there is also the generation of an alarm such as an audible tone. The trigger thresholds may vary for the different processing outcomes of direct pressure measurements and alarms and other actions. Also, other values of the waveform of V can be determined instead of or in addition to its maximum and minimum, such as its mean value.

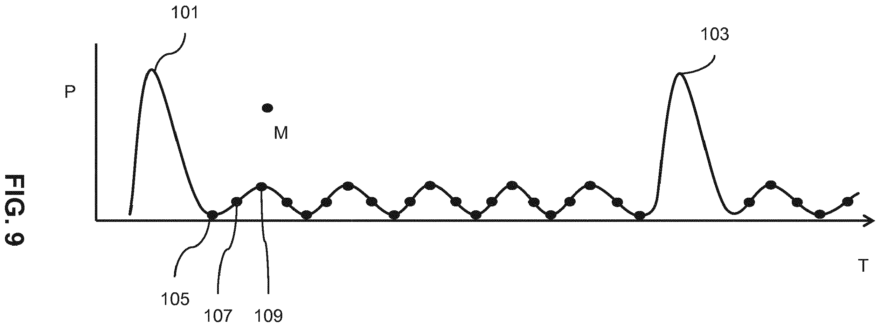

As shown in Fig. 9, which is a graph of cuff pressure P against time T, the regular direct measurements using the pressure cuff are taken at times labelled 101 and 103. Between these times, blood pressure is monitored whilst the cuff pressure is modulated at a low level. The instants at which maximum and minimum velocity values are taken and stored are shown by dots M, at the same phases of the modulation cycle. In this example, they are taken at the minimum cuff pressure value 105, the mean pressure value 107 and the maximum pressure value 109 of each cycle.

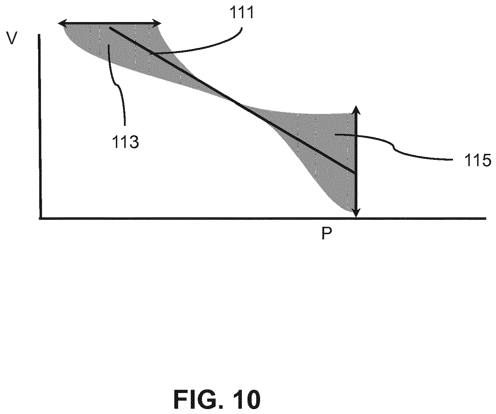

As illustrated in Fig. 10, in which the true measure is represented by the line 111, the amplitude of the slow pressure modulation is a compromise between accuracy (high amplitude modulation, thus including periods at relatively high pressure) and patient comfort and sufficient blood circulation (low or zero amplitude). At low cuff pressure, the pressure measurement is relatively inaccurate, shown by an uncertainty band 113, since it is influenced by the cuff strap pressure, the patient's movements and external pressures. The Doppler reading even at low cuff pressure is relatively accurate due to the high signal to noise ratio (maximum blood flow). At higher cuff pressures, the external pressure influences are relatively low and the pressure reading of the cuff is predominantly determined by the cuff-arm contact. However, the Doppler reading is less accurate, shown by an uncertainty band 115, due to the lower blood speeds through the artery. Therefore, measuring the Doppler signal at several different cuff pressures improves the overall accuracy, since the data can be fitted and extrapolated to model data, as illustrated in Fig. 10.

A patient needing blood pressure monitoring is fitted with the cuff 1 over the Doppler transducer, placed over the arm or ankle by a trained nurse. The cuff then starts inflating and a first blood pressure measurement is obtained. Afterwards, the cuff remains in position and processed Doppler data from which vital signs are derived are shown at the cuff monitor 17 and also at a monitor (not shown) connected to the control unit 7 or the external system 31, during the modulation cycles of the cuff. The control system controls the scheduling of repeated direct blood pressure measurements, and the generation of alerts and scheduling information as shown in Figs. 4 to 6.

In both embodiments described, the correlation between blood flow velocity and blood pressure is recalibrated whenever a direct cuff-based measurement is made. However, this may not be essential. It may be sufficient to recalibrate less frequently, for example either every fixed time period, such as 12 or 24 hours, or every time a recalibration is manually instructed, or each time the system is turned on. It may also be sufficient to use predetermined calibration settings when only sudden blood pressure changes are of interest.

Although in the embodiments described the arterial pressure is sensed by a pressure sensor connected to the air pump that inflates the cuff, so that in the steady state it senses the contact pressure between the cuff and the skin above the artery, this is not essential. The arterial blood pressure could be measured independently using a separate sensor or using another technique such as the pulse-wave velocity technique described above. The scheduled direct blood pressure measurements could be taken using the more accurate mercury manometer connected to the cuff, and the results fed into the control unit 7 for calibrating the model, look-up table, etc.

The invention is especially useful for monitoring the blood pressure of 'at risk' patients in a hospital situation. Perioperative and low-acuity monitoring of patients at risk of hemodynamic instability or deterioration can be performed at minimal disturbance of the patient. It may also be a valuable addition to conventional ambulatory monitoring systems.

The cuff-based pressure measurements described above involve a deflation technique. However, the invention is not limited to this, and instead it could involve linear inflation technology, i.e. the taking of blood pressure measurements whilst inflating the cuff. The systolic pressure is measured as the cuff is inflated, at the point occlusion is detected. It has been shown that this NIBP technique reduces the measurement duration and also can reduce the target maximum inflation pressure. It is particularly advantageous over the deflation technique when the systolic pressure keeps changing, because the deflation method could fail to reach the necessary pressure for occlusion, and could then require repetition with a higher target, if the systolic pressure has risen since the last measurement; and it could overshoot the required occlusion pressure and cause unnecessary delay deflating, if the systolic pressure has dropped since the last measurement.

As discussed above, embodiments make use of a control unit. The control unit can be implemented in numerous ways, with software and/or hardware, to perform the various functions required. A processor is one example of a controller which employs one or more microprocessors that may be programmed using software (e.g., microcode) to perform the required functions. A controller may however be implemented with or without employing a processor, and also may be implemented as a combination of dedicated hardware to perform some functions and a processor (e.g., one or more programmed microprocessors and associated circuitry) to perform other functions.

Examples of controller components that may be employed in various embodiments of the present disclosure include, but are not limited to, conventional microprocessors, application specific integrated circuits (ASICs), and field-programmable gate arrays (FPGAs).

In various implementations, a processor or controller may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor or controller.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope.

## Claims

1. A method of non-invasive, continuous monitoring of arterial blood pressure of a subject using a pressure cuff (1) placed around a limb of the subject, comprising:
(91) measuring systolic and diastolic blood pressures whilst inflating and/or deflating the cuff between pressures at which arterial blood flow is impeded and at which it is unimpeded;
(93) measuring the arterial blood flow velocity using Doppler ultrasound imaging, whilst measuring the systolic and diastolic pressures;
(94) processing and correlating the measurements of blood pressure and blood flow velocity and storing a representation of their correlation;
(95) setting the cuff pressure at a low level which is below diastolic blood pressure, and (97) monitoring the arterial blood flow velocity waveform, using Doppler ultrasound imaging;
(99) determining from the monitoring whether a predetermined trigger condition applies such that a pressure cuff-based measurement of blood pressure is required; and, if so,
repeating the step (91) of measuring systolic and diastolic blood pressures.

2. A method according to claim 1, further comprising after repeating the step of measuring systolic and diastolic blood pressures, repeating the arterial blood flow measuring, processing and correlating, and storing steps, so as to recalibrate the correlation.

3. A method according to claim 1 or 2, comprising using in the step of measuring the arterial blood flow velocity an ultrasonic transducer (3) in the cuff to measure the arterial blood flow velocity.

4. A method according to any preceding claim, comprising slowly modulating the low cuff pressure in the setting and monitoring step, preferably with a modulation period comprising a multiplicity of heartbeat periods, and using the Doppler ultrasound imaging to measure the arterial blood flow velocity waveform as it varies with this modulation.

5. A method according to claim 4, comprising monitoring the variation, over successive periods of the slow modulation of the low cuff pressure, of the peak and/or minimum blood velocity in a heartbeat, and using the result of this monitoring to determine whether the predetermined trigger condition applies.

6. A method according to claim 5, comprising monitoring the variation of the peak and/or minimum blood flow velocities occurring at the same phase of successive periods of the slow modulation.

7. A method according to any preceding claim, comprising scheduling the direct blood pressure measurements performed in the measuring steps to occur regularly during each day and night, at intervals exceeding an hour, preferably at intervals of three hours or more, and modifying that schedule either by eliminating some measurements or by adding previously unscheduled measurements in response to the determination whether a direct measurement is required.

8. Apparatus for non-invasive, continuous monitoring of arterial blood pressure of a subject, comprising a pressure cuff (1) arranged to be placed around a limb of the subject, means (23) for measuring the pressure applied to the pressure cuff, and a Doppler ultrasound imaging system (3) for imaging blood flow in the limb beneath or adjacent to the cuff, and a cuff controller (7) configured to:
measure systolic and diastolic blood pressures using the pressure measuring means whilst causing the inflation and/or deflation of the cuff between pressures at which arterial blood flow is impeded and at which it is unimpeded;
measure the arterial blood flow velocity using the Doppler ultrasound imaging system, whilst measuring the systolic and diastolic pressures;
process and correlate the measurements of blood pressure and blood flow velocity and store a representation of their correlation;
set the cuff pressure at a low level which is below diastolic blood pressure, and monitor the arterial blood flow velocity waveform, using the Doppler ultrasound imaging system;
determine from the monitoring whether a predetermined trigger condition applies such that a pressure cuff-based measurement of blood pressure is required; and, if so,
repeat the measurement of systolic and diastolic blood pressures using the pressure measuring means.

9. Apparatus according to claim 8, wherein the cuff controller (7) is further configured, after repeating the step of measuring systolic and diastolic blood pressures, to repeat the arterial blood flow measuring, processing and correlating, and storing steps, so as to recalibrate the correlation.

10. Apparatus according to claim 8 or 9, wherein the cuff controller (7) is configured to measure the arterial blood flow velocity using an ultrasonic transducer (3) in the cuff.

11. Apparatus according to any one of claims 8 to 10, the cuff controller (7) configured to slowly modulate the low cuff pressure with a modulation period preferably comprising a multiplicity of heartbeat periods, and use the Doppler ultrasound imaging to measure the arterial blood flow velocity waveform as it varies with this modulation.

12. Apparatus according to claim 11, wherein the cuff controller (7) is configured to monitor the variation, over successive periods of the slow modulation of the low cuff pressure, of the peak and/or minimum blood velocity in a heartbeat; and use the result of this monitoring to determine whether the predetermined trigger condition applies.

13. Apparatus according to claim 12, wherein the cuff controller (7) is configured to monitor the variation of the peak and/or minimum blood flow velocities occurring at the same phase of successive periods of the slow modulation.

14. Apparatus according to any preceding claim, wherein the cuff controller (7) is configured to schedule the direct blood pressure measurements of the measuring steps to occur regularly during each day and night, at intervals exceeding an hour, preferably at intervals of three hours or more, and modify that schedule either by eliminating some measurements or by adding previously unscheduled measurements in response to the determination in the determining step whether a direct measurement is required.

15. A computer program comprising computer program code means which is adapted, when said program is run on a computer of a cuff controller, to implement the method of any one of claims 1 to 7.
